# EUROPÄISCHE PATENTSCHRIFT

(11) **EP 1 728 522 B1**
(45) Veröffentlichungstag und Bekanntmachung des Hinweises auf die Patenterteilung: **22.05.2013**
(21) Anmeldenummer: 05011763.9
(22) Anmeldetag: 31.05.2005
(51) Int. Cl.: A61L 2/26, A61F 2/06, A61L 31/02, A61L 31/08, A61L 31/12

(54) **Beschichtete Stents**
Coated Stents
Prothese Endovasculaire Revêtue

(43) Veröffentlichungstag der Anmeldung: 06.12.2006
(73) Patentinhaber: Axetis AG, 6300 Zug (CH)
(72) Erfinder: Civelli, Carlo, 8023 Zürich (CH)
(74) Vertreter: Bremi, Tobias Hans

(56) Entgegenhaltungen:
- EP-A- 1 405 647
- WO-A-00/64506
- WO-A-01/15751
- WO-A-03/000308
- DE-A1- 10 123 441
- US-A- 4 437 192
- US-A- 6 136 156

## Beschreibung

Die vorliegende Erfindung ist auf einen Gefäßstent, beispielsweise zur Implantation in Blutgefäße eines Körpers, gerichtet.

Sogenannte "Stents" werden zum Zweck der Offenhaltung von Gefäßen wie Blutgefäßen (bei Arteriosklerose) in die Okklusions-gefährdeten Gefäße eingesetzt. Dies kann entweder mittels eines Katheters geschehen oder durch operative Eröffnung des Gefäßes, ggfls. Ausräumen und Implantieren des Stents. Stents sind im allgemeinen schlauchförmige, rohrtörmige Strukturen, beispielsweise Gewebeschläuche oder röhrenartige poröse Strukturen, die sich an die Innenwandung eines Gefäßes anschmiegen und einen freien Strömungsquerschnitt offen halten, durch welches das Blut in dem Blutgefäß frei fließen kann.

Weitere Einsätze von Stents sind in Gallenwegen, Luftröhre oder Speiseröhre. So werden Stents beispielsweise in der Behandlung von Karzinomen dazu eingesetzt, die Verengungen bei den Atemwegen, Gallenwegen oder der Speiseröhre nach einer ertolgten Dehnung zu begrenzen.

Stents bestehen häufig aus Röhrchen mit netzförmiger Wandung, die einen kleinen Durchmesser aufweisen und dadurch leicht mittels eines Katheters an den Zielort gebracht werden können, wo sie mit Hilfe eines Ballons (Ballonkatheter) im Gefäß durch Dehnung der netzförmigen Wand des Stents auf das notwendige Lumen und damit den notwendigen Durchmesser zur Abstützung des Gefäßes expandiert werden können.

Es ist bekannt, Stents mit Kunststoffen, wie etwa Polytetrafluorethylen (PTFE;Teflon® ) zu beschichten.

Vorbekannten Stents ist jedoch das Problem zueigen, dass sie aufgrund ihrer spezifischen Oberfläche und ihrer Gitterstruktur oft von körpereigenen Zellen durchwachsen bzw. umwachsen werden, was längerfristig wiederum zu einer emeuten Okklusion des mit einem Stent abgesicherten Gefäßes führen kann. Hier ist es schwierig, den gewünschten Kompromiss zwischen Offenhalten des Gefäßes und harmonischer Integration des Stents in den Organismus zu finden. Auch sind herkömmliche Stentbeschichtungen nicht immer flexibel genug, um die Bewegungen des Stents beim Implantieren und Expandieren mitzumachen, was zu Beschädigungen an der Beschichtung führen kann. Auch hat sich gezeigt, dass sich zwischen den Stentwerkstoffen und dem Blut beziehungsweise anderem Gewebe ein elektronisches Potential aufbauen kann, wobei solche Potentiale die Eigenschaften der Blutinhaltsstoffe in der Grenzschicht zu den Stentwerkstoffen nachteilig verändern können und dadurch zu unkontrollierten Ablagerungen wie Plaques etc. führen können. Diese Probleme finden sich teilweise auch bei anderen medizinischen Implantaten mit ähnlichen Anforderungen.

Es besteht daher weiterhin ein Bedarf an medizinischen Implantaten, wie etwa Stents, die eine problemlose Implantation in den Körper eines Patienten gestatten.

Diese Aufgabe wird gelöst durch die Bereitstellung eines Gefäßstents gemäß dem unabhängigen Patentanspruch 1 und eines Verfahrens zur Herstellung eines beschichteten Gefäßstents gemäß dem unabhängigen Patentanspruch 20. Weitere vorteilhafte Ausgestaltungen, Aspekte und Details der vorliegenden Erfindung ergeben sich aus den abhängigen Patentansprüchen und der Beschreibung.

Der Erfindung liegt die Idee zugrunde, einen Gefäßstent mit einer siliziumdioxidhaltigen Beschichtung, also einer glasähnlichen Beschichtung, zu versehen.

Das Siliziumdioxid kann in amorpher oder kristalliner oder halbkristalliner Form vorliegen.

Die Eigenschaften der Beschichtung werden durch zumindest eine Beimischung, die in der Beschichtung enthalten ist, modifiziert, wobei die Beimischung ausgewählt ist aus Titanoxid, Calciumverbindungen, Natriumoxid, Germaniumoxid, Magnesiumoxid, Selenoxid und Hydroxiden, insbesondere Hydroxiden der vorgenannten Metalle. Ein besonders bevorzugte Beimischungen ist Titanoxid.

Der Anteil der Beimischung an der Gesamtmenge der Beschichtung kann vorzugsweise 0,5 bis 50 Gew.-% betragen.

Um über die gesamte Oberfläche des Gefäßstents, die gewünschte Oberflächeneigenschaften zu bewahren, wird es bevorzugt, dass die Beschichtung im wesentlichen porenfrei ist.

Bei bestimmten Ausführungsformen kann es jedoch ebenfalls bevorzugt werden, dass die Beschichtung Poren für eine Funktionalisierung mit weiteren Substanzen, die nach der eigentlichen Beschichtung auf die Beschichtung aufgebracht werden und sich in den Poren ablagem, aufweist. Dementsprechend kann die erfindungsgemäße Beschichtung einen zusätzlichen, auch nur partiell oder punktuell vorhandenen Funktionalisierungsauftrag aufweisen. Ein solcher Auftrag kann der medizinischen Zweckbestimmung des medizinischen lmplantates entsprechen und eine Beeinflussung des Wachstums von umgebendem Gewebe, eine Abtötung unerwünschten Gewebes, den Aufbau einer Beziehung zwischen medizinischem Implantat und Gewebe, etc. beinhalten. Der Funktionalisierungsauftrag kann beispielsweise zumindest ein Medikament und/oder zumindest ein Zellgift enthalten.

Ein großer Vorteil der erfindungsgemäßen medizinischen Implantate ist darin zu sehen, dass die Beschichtung extrem dünn aufgebracht wird, nämlich im Nano-Bereich, also im Bereich einiger Atomschichten, was es gestattet, bei der Herstellung des medizinischen Implantats im wesentlichen die Endabmessungen einstellen zu können, ohne auf möglicherweise nicht exakt vorhersehbare Dimensionierungsänderungen durch die Beschichtung Rücksicht nehmen zu müssen. Die Dicke der erfindungsgemäßen Beschichtung beträgt 0,1 bis 1000 nm,. Maßgebend bei der Wahl der Schichtdicke ist die Anforderung, dass bei der Expansion des Implantates im Körper die Beschichtung nicht beschädigt wird und keine zusätzlichen Poren entstehen.

Die Beschichtung kann in einem einzelnen Schritt aufgetragen werden, und somit eine einlagige Schicht bilden, kann jedoch in einer bevorzugten Ausführungsform auch aus mehreren, sukzessive aufgebrachten Schichten bestehen. Bei mehrlagigen Verfahren kann die Zusammensetzung jeder einzelnen Schicht individuell festgelegt werden.

Der Gefäßstent kann für ein Blutgefäß, einen Gallengang, die Speiseröhre oder die Luftröhre bestimmt sein, wobei er bei verschiedenen Tierarten, wie Menschen, Haustieren, und Nutztieren verwendet werden kann.

Der Träger ist vorzugsweise aus einem schwer abbaubaren Material aufgebaut, wobei unter "schwer abbaubar" eine Eigenschaft zu verstehen ist, bei der das Material nach Implantation in einen Körper für zumindest ein Jahr keine sichtbaren Abbauerscheinungen zeigt.

Der Träger kann für medizinische Implantate, insbesondere Gefäßstents übliche Materialien, wie Carbon, PTFE, Dacron, Metall-Legierungen oder PHA umfassen, wobei insbesondere Stahllegierungen bevorzugte Materialien sind.

Die erfindungsgemäß für den Träger verwendbaren Metalllegierungen werden vorzugsweise ausgewählt aus Edelstählen.

Ein weiteres bevorzugtes Material für den Träger ist ein Formgedächtnismetall, insbesondere Nickel-Titanlegierungen, die aufgrund ihrer Fähigkeiten zur eigenständigen Formveränderung bei Stents Einsatz finden.

Schließlich ist die Erfindung in einem weiteren Aspekt ebenfalls auf ein Verfahren zur Herstellung eines Gefäßstents gerichtet, welches die Schritte aufweist:
- Bereitstellen eines die Grundstruktur bildenden Trägers; und
- Aufbringen einer Siliziumdioxid enthaltender Beschichtung gemäß dem Anspruch 1 mittels eines Plasmabeschichtungsverfahrens.

Alles bezüglich dem Gefäßstent Gesagte gilt sinngemäß auch für das erfindungsgemäße Verfahren und umgekehrt, so dass wechselweise Bezug genommen wird.

Um die bei bestimmten Ausführungsformen gewünschten Poren zur Aufnahme von Funktionalisierungsmitteln zu erhalten, wird es weiterhin bevorzugt, dass das Verfahren den Schritt des Erzeugens von Poren in der Beschichtung mittels eines Neutronenbeschusses umfasst. Hiertür können Neutronenquellen wie beispielsweise Teilchenbeschleuniger verwendet werden. Eine weitere Variante zur Erzeugung von Funktionsporen besteht darin, die Poren mittels Laserlicht herzustellen.

Die vorliegende Erfindung stellt eine Beschichtung für Gefäßstents, dar, die aufgrund ihrer inerten, glasartigen Oberfläche mit Siliziumdioxid einen Bewuchs mit Zellen des Körpers bzw. einer Anhaftung solcher Zellen, weitgehend verhindert, die aufgrund ihrer Härte einer Beschädigung beim Einbringen des Implantats in den Körper entgegenwirkt und damit die Handhabung vereinfacht, die aufgrund der Dünne der Beschichtung eine einfachere Gestaltung des Implantats gestattet, eine verminderte Reibung durch geringere Rauhigkeitswerte und damit eine kleinere Belastung für Blutkomponenten und niedriger Koagelbildung aufweist und bei der es auch nach längerer Verbleibdauer im Körper keinerlei Abbau der Beschichtung gibt.

## Patentansprüche

1. Medizinisches Implantat, aufweisend einen eine Grundstruktur bildenden Träger und eine auf zumindest Teile des Trägers aufgebrachte Beschichtung,
• wobei die Beschichtung Siliziumdioxid aufweist;
• wobei die Beschichtung eine Beimischung enthält, die ausgewählt ist aus Titanoxid, Kalzium-Verbindungen, Natriumoxid, Germaniumoxid, Magnesiumoxid, Selenoxid, und Hydroxiden, insbesondere Hydroxiden der vorgenannten Metalle, und Mischungen derselben;
• wobei die Dicke der Beschichtung 0,1 bis 1000 nm beträgt; und
• wobei das medizinische Implantat ein Gefäßstent ist.

2. Medizinisches Implantat gemäß Anspruch 1, **dadurch gekennzeichnet, dass** ein Anteil der Beimischung an der Gesamtmenge der Beschichtung 0,5 bis 50 Gew.-% beträgt.

3. Medizinisches Implantat gemäß Anspruch 1 oder 2, **dadurch gekennzeichnet, dass** die Beschichtung im wesentlichen porenfrei ist.

4. Medizinisches Implantat gemäß einem der Ansprüche 1 bis 2 **dadurch gekennzeichnet, dass** die Beschichtung Poren für eine Funktionalisierung aufweist.

5. Medizinisches Implantat gemäß einem der Ansprüche 1 bis 4, **dadurch gekennzeichnet, dass** die Beschichtung einen zusätzlichen Funktionalisierungsauftrag enthält.

6. Medizinisches Implantat gemäß Anspruch 5, **dadurch gekennzeichnet, dass** der Funktionalisierungsauftrag zumindest ein Medikament, und/oder zumindest ein Zellgift aufweist.

7. Medizinisches Implantat gemäß einem der Ansprüche 1 bis 6, **dadurch gekennzeichnet, dass** die Beschichtung aus mehreren, sukzessive aufgebrachten Schichten besteht.

8. Medizinisches Implantat gemäß einem der Ansprüche 1 bis 7, **dadurch gekennzeichnet, dass** der Träger aus einem schwer abbaubaren Material aufgebaut ist.

9. Medizinisches Implantat gemäß einem der Ansprüche 1 bis 8, **dadurch gekennzeichnet, dass** der Träger Karbon, PTFE, Dacron (Polyethylenterephthalat, PET), Metalllegierungen, PHA umfasst.

10. Medizinisches Implantat gemäß Anspruch 9, **dadurch gekennzeichnet, dass** der Träger Eisenlegierungen umfasst.

11. Medizinisches Implantat gemäß Anspruch 10, **dadurch gekennzeichnet, dass** die Eisenlegierung ein Edelstahl ist.

12. Medizinisches Implantat gemäß Anspruch 8, **dadurch gekennzeichnet, dass** der Träger ein Formgedächtnismetall, insbesondere Nickel-Titanlegierungen, umfasst.

13. Medizinisches Implantat gemäß einem der Ansprüche 1 bis 12, **dadurch gekennzeichnet, dass** der Gefäßstent für ein Blutgefäß, einen Gallengang, die Speiseröhre oder die Luftröhre bestimmt ist.

14. Verfahren zur Herstellung eines beschichteten medizinischen Implantats nach einem der Ansprüche 1 bis 13, die Schritte aufweisend:
• Bereitstellen eines eine Grundstruktur bildenden Trägers; und
• Aufbringen einer Siliziumdioxid enthaltender Beschichtung mit einer Dicke von 0,1 bis 1000 nm mittels eines Plasmabeschichtungsverfahrens.

15. Verfahren gemäß Anspruch 14, **dadurch gekennzeichnet, dass** es weiterhin das Erzeugen von Poren in der Beschichtung mittels Neutronenbeschuss oder Laser umfasst.

## Claims

1. A medical implant, comprising a substrate that forms a basic structure and a coating applied to at least sections of the substrate,
- wherein the coating comprises silicon dioxide;
- wherein the coating comprises at least one ingredient selected from the group consisting of titanium oxide, calcium compounds, sodium oxide, germanium oxide, magnesium oxide, selenium oxide, and hydroxides, especially hydroxides of the aforementioned metals, and mixtures of these;
- wherein the thickness of the coating is from 0.1 to 1000 nm; and
- wherein the medical implant is a vascular stent.

2. The medical implant according to claim 1, **characterized in that** the coating comprises the at least one ingredient in an amount of approximately 0.5 to 50% by weight of the coating.

3. The medical implant according to claim 1 or 2, **characterized in that** the coating is essentially free of pores.

4. The medical implant according to one of claims 1 to 2, **characterized in that** the coating comprises pores for a functionalization.

5. The medical implant according to one of claims 1 to 4, **characterized in that** the coating has an additional functionalization coat.

6. The medical implant according to claim 5, **characterized in that** the functionalization coat contains at least one medicament and/or at least one cell poison.

7. The medical implant according to one of claims 1 to 6, **characterized in that** the coating consists of several successively applied layers.

8. The medical implant according to one of claims 1 to 7, **characterized in that** the substrate comprises a difficult-to-degrade material.

9. The medical implant according to one of claims 1 to 8, **characterized in that** the substrate comprises a material selected from the group consisting of carbon, PTFE, Dacron (Polyethylenterephthalat, PET), metal alloys and PHA.

10. The medical implant according to claim 9, **characterized in that** the substrate comprises iron alloys.

11. The medical implant according to claim 10, **characterized in that** the iron alloy is a precious metal.

12. The medical implant according to claim 8, **characterized in that** the substrate comprises a shape memory metal, especially nickel titanium alloys.

13. The medical implant according to one of claims 1 to 12, **characterized in that** the vascular stent is intended for a blood vessel, a bile duct, the esophagus or the trachea.

14. Method for the production of a coated medical implant according to one of claims 1 to 13, comprising the following steps:
- provision of a substrate forming a basic structure; and
- application of a coating comprising silicon dioxide by means of a plasma coating method, the coating having a thickness of 0.1 to 1000 nm.

15. The method according to claim 14, **characterized in that** it further comprises the production of pores in the coating by means of neutron bombardment or laser light.

## Revendications

1. Implant médical, présentant un support formant une structure de base et un revêtement appliqué sur au moins des parties du support,
- dans lequel le revêtement présente du dioxyde de silicium,
- dans lequel le revêtement contient une adjonction qui est sélectionnée parmi l'oxyde de titane, des liaisons calciques, l'oxyde de sodium, l'oxyde de germanium, l'oxyde de magnésium, l'oxyde de sélénium et des hydroxydes, en particulier des hydroxydes des métaux nommés ci-avant et des mélanges de ceux-ci ;
- dans lequel l'épaisseur du revêtement est de 0,1 à 1000 nm ; et
- dans lequel l'implant médical est une prothèse endovasculaire.

2. Implant médical selon la revendication 1, **caractérisé en ce qu'**une part de l'adjonction sur la quantité totale du revêtement est de 0,5 à 50% en poids.

3. Implant médical selon la revendication 1 ou 2, **caractérisé en ce que** le revêtement est essentiellement exempt de pores.

4. Implant médical selon l'une des revendications 1 à 2, **caractérisé en ce que** le revêtement présente des pores pour une fonctionnalisation.

5. Implant médical selon l'une des revendications 1 à 4, **caractérisé en ce que** le revêtement comporte une application de fonctionnalisation supplémentaire.

6. Implant médical selon la revendication 5, **caractérisé en ce que** l'application de fonctionnalisation supplémentaire présente au moins un médicament et/ou une substance toxique pour les cellules.

7. Implant médical selon l'une des revendications 1 à 6, **caractérisé en ce que** le revêtement est composé d'une pluralité de couches appliquées successivement.

8. Implant médical selon l'une des revendications 1 à 7, **caractérisé en ce que** le support est fabriqué dans un matériau difficilement dégradable.

9. Implant médical selon l'une des revendications 1 à 8, **caractérisé en ce que** le revêtement comprend du carbone, du PTFE, du Dacron (polytéréphtalate d'éthylène, PET), des alliages de métaux, du PHA.

10. Implant médical selon la revendication 9, **caractérisé en ce que** le support comprend des alliages ferreux.

11. Implant médical selon la revendication 10, **caractérisé en ce que** l'alliage ferreux est un acier inoxydable.

12. Implant médical selon la revendication 8, **caractérisé en ce que** le support comprend un métal à mémoire de forme, en particulier des alliages nickel-titane.

13. Implant médical selon l'une des revendications 1 à 12, **caractérisé en ce que** la prothèse endovasculaire est destinée à un vaisseau sanguin, à un canal biliaire, à l'oesophage ou à la trachée.

14. Procédé de fabrication d'un implant médical revêtu selon l'une des revendications 1 à 13, présentant les étapes de :
- préparation d'un support formant la structure de base ; et
- application d'un revêtement contenant du dioxyde de silicium d'une épaisseur de 0,1 à 1000 nm via un procédé de revêtement au plasma.

15. Implant médical selon la revendication 14, **caractérisé en ce qu'**il comprend en outre la fabrication de pores dans le revêtement via un tir de neutrons ou un laser.
